# EUROPÄISCHE PATENTSCHRIFT

(11) **EP 0 495 218 B1**
(45) Veröffentlichungstag und Bekanntmachung des Hinweises auf die Patenterteilung: **14.06.1995**
(21) Anmeldenummer: 91121660.4
(22) Anmeldetag: 18.12.1991
(51) Int. Cl.: C07C 273/18

(54) **Verfahren zur Herstellung N-mono- oder N,N-disubstituierter Harnstoffe**
Process for the preparation of N-mono or N,N-disubstituted ureas
Procédé pour la préparation d'urées N-mono ou N,N-disubstituées

(30) Priorität: 18.01.1991 AT 111/91
(43) Veröffentlichungstag der Anmeldung: 22.07.1992
(73) Patentinhaber: Chemie Linz GmbH, A-4021 Linz (AT)
(72) Erfinder: Rössler, Markus, A-4020 Linz (AT); Stern, Gerhard, Dr. Dipl.-Ing., A-4180 Sonnberg (AT); Müllner, Martin, Dr. Dipl.-Ing., A-4020 Linz (AT)
(74) Vertreter: Kunz, Ekkehard, Dr.

(56) Entgegenhaltungen:
- CH-A- 318 184
- DE-A- 3 928 566

## Beschreibung

Die vorliegende Erfindung betrifft ein Verfahren zur Herstellung N-mono- oder N,N-disubstituierter Harnstoffe durch Umsetzung von Ammoniumisocyanat mit primären oder sekundären Aminen.

Die Herstellung von N-mono- oder N,N-disubstituierten Harnstoffen durch Umsetzung von Isocyansäure, die z.B. mittels Säurezugabe aus Kalium- oder Natriumisocyanat freigesetzt wird, mit primären oder sekundären Aminen ist bekannt und beispielsweise in Houben-Weyl, Methoden der organischen Chemie, Erweiterungs- und Folgebände zur vierten Auflage, Band E4, Seiten 362 bis 364 beschrieben. Ein ausführliches Beispiel für eine derartige Umsetzung ist in Organic Syntheses, Vol 31 (1951), Seiten 9 bis 11 geoffenbart, wo die Umsetzung von p-Bromanilin mit Natriumisocyanat in Eisessig/Wasser zu p-Bromphenylharnstoff beschrieben ist. In EP-A-0 333 350 ist die Herstellung aromatischer Bis-dialkylharnstoffe durch Reaktion eines aromatischen Diamins mit Isocyansäure, die durch Ansäuren von Kalium-, Natrium- oder Silberisocyanat freigesetzt wird, beschrieben.

Es wurde nun gefunden, daß primäre oder sekundäre Amine nicht nur mit Isocyansäure, sondern auch mit einem Salz der Isocyansäure, nämlich mit Ammoniumisocyanat zu N-mono- oder N,N-disubstituierten Harnstoffen umgesetzt werden können. Unerwarteterweise muß die Isocyansäure aus dem Ammoniumisocyanat dabei nicht durch Zugabe von Säure in Freiheit gesetzt werden.

Gegenstand der Erfindung ist daher ein Verfahren zur Herstellung N-mono- oder N,N-disubstituierter Harnstoffe, das dadurch gekennzeichnet ist, daß Ammoniumisocyanat in einem Verdünnungsmittel mit einem primären oder sekundären Amin umgesetzt wird.

Ammoniumisocyanat entsteht beispielsweise beim schnellen Vergasen von Harnstoff und Abschrecken der Reaktionsgase, etwa nach US-A-2.712.491 oder beim Einsprühen von geschmolzenem Harnstoff in gasförmiges Ammoniak bei hohen Temperaturen und Abschrecken der Reaktionsmischung, etwa nach US-A-2.712.492 und kann auf diese Weise hergestellt werden.

Unter primären und sekundären Aminen sind solche Verbindungen zu verstehen, die eine oder mehrere Aminogruppen aufweisen. Sie können gegebenenfalls durch weitere, unter den Reaktionsbedingungen inerte Gruppen substituiert sein. Beispiele hiefür sind aliphatische, cycloaliphatische oder cyclische Amine, wie Methylamin, Ethylamin, Hexylamin, Hexadecylamin, Isopropylamin, Isobutylamin, Isooctylamin, Methylethylamin, Cyclohexylamin, Pyrrolidin, Pyrrol, Piperidin, Morpholin oder Dimethylamin, Diethylamin, Diisopropylamin, Ethylendiamin, Hexamethylendiamin, 4,4'-Diaminodicyclohexylmethan oder aromatische Amine, wie Anilin, Nitroaniline, Chloraniline, Tolylamine, Benzylamin, Naphthylamine, Phenylendiamine, Toluylendiamine, 4,4'-Diaminodiphenylmethan.

Zur Durchführung des erfindungsgemäßen Verfahrens wird entweder Ammoniumisocyanat in einem unter den Reaktionsbedingungen inerten Verdünnungsmittel vorgelegt, gerührt und mit einem primären oder sekundären Amin versetzt oder das primäre oder sekundäre Amin wird in einem unter den Reaktionsbedingungen inerten Verdünnungsmittel, oder, falls das Amin flüssig ist, ohne zusätzliches weiteres Verdünnungsmittel vorgelegt, wobei das Amin gleichzeitig auch als Verdünnungsmittel dienen kann, gerührt und mit Ammoniumisocyanat versetzt.

Als inerte Verdünnungsmittel kommen beispielsweise aliphatische Kohlenwasserstoffe, wie Pentan, Hexan, Heptan, aromatische Kohlenwasserstoffe, wie Benzol, Toluol, Xylol, halogenierte aliphatische Kohlenwasserstoffe, wie Methylenchlorid, Chloroform, Tetrachlorkohlenstoff, Ethylchlorid, Ethylenchlorid, halogenierte aromatische Kohlenwasserstoffe, wie Chlorbenzol, Trichlorbenzole, Ether, wie Diethylether, Diisopropylether, Dibutylether, Ethylmethylether, Dioxan, Tetrahydrofuran, Dimethoxyethan, Carbonsäureamide, wie Dimethylformamid, N-Methylpyrrolidon, Alkohole wie Methanol, Ethanol, Diisopropylalkohol, sowie Wasser oder Mischungen solcher Verdünnungsmittel in Frage. Bevorzugt sind aliphatische und aromatische Kohlenwasserstoffe, halogenierte, aliphatlsche oder aromatische Kohlenwasserstoffe, Ether, Alkohole sowie Wasser, besonders bevorzugt sind n-Hexan, Chloroform, Chlorbenzol, Dioxan sowie Wasser.

Ammoniumisocyanat kann als solches, oder zusammen mit einem unter den Reaktionsbedingungen inerten, wie oben beschriebenen flüssigen Verdünnungsmittel, das primäre oder sekundäre Amin als solches, gasförmig oder flüssig, oder zusammen mit einem unter den Reaktionsbedingungen inerten wie oben beschriebenen gasförmigen oder flüssigen Verdünnungsmittel zugegeben werden.

Die Zugabe erfolgt bei Temperaturen von etwa -20 °C bis zum Siedepunkt des verwendeten Verdünnungsmittels, bevorzugt bei Temperaturen von etwa -10 °C bis Raumtemperatur, besonders bevorzugt bei Raumtemperatur.

Das Amin wird dabei in äquivalenter Menge oder in einem Überschuß zum Ammoniumisocyanat zugegeben. Es kann aber auch zweckmäßig sein, Ammoniumisocyanat im Überschuß zuzusetzen, um den Ablauf der Reaktion zu verbessern. Bis auf den Fall, wo das Amin gleichzeitig als Verdünnungsmittel eingesetzt wird, wird Ammoniumisocyanat und das Amin bevorzugt äquivalent eingesetzt.

Nach beendeter Zugabe des Amins oder des Ammoniumisocyanats wird zur Vervollständigung der Reaktion bei Temperaturen bis zur Rückflußtemperatur des verwendeten Verdünnungsmittels, bevorzugt erst bei Raumtemperatur und anschließend bei Temperaturen bis zur Rückflußtemperatur des verwendeten Verdünnungsmittels nachgerührt. Gegebenenfalls erfolgt die Reaktion auch unter Druck, wobei Drücke bis zu 20 bar angewendet werden können.

Nach dem Abkühlen kristallisiert die gebildete Verbindung aus dem Verdünnungsmittel aus und wird abfiltriert, oder das Verdünnungsmittel und gegebenenfalls flüchtige Bestandteile der Reaktionsmischung werden verdampft. Gegebenenfalls kann auf übliche Weise, wie z.B. durch Umkristallisation, Destillation, Chromatographie, eine weitere Reinigung erfolgen. Sollte sich als Nebenprodukt Harnstoff ausgebildet haben, was vorkommen kann, kann der Harnstoff je nach der Natur des verwendeten Verdünnungsmittels als Feststoff ausfallen und gegebenenfalls durch Abfiltrieren, Umkristallisieren oder chromatographische Methoden auf übliche Weise entfernt werden.

In einer bevorzugten Ausführungsform wird Ammoniumisocyanat in einem unter den Reaktionsbedingungen inerten Verdünnungsmittel gelöst oder suspendiert und bei Raumtemperatur unter Rühren mit der äquivalenten Menge primären oder sekundären Amins, verdünnt mit dem gleichen inerten Verdünnungsmittel, versetzt. Nach beendeter Zugabe wird zur Vervollständigung der Reaktion bei Raumtemperatur nachgerührt und bis auf Rückflußtemperatur des verwendeten Verdünnungsmittels erhitzt. Nach dem Abkühlen wird der ausgefallene Niederschlag abgesaugt und gegebenenfalls umkristallisiert. Bei Verwendung von Wasser als Verdünnungsmittel kann das Reaktionsprodukt auch direkt aus der Reaktionslösung nach Verdünnung mit Wasser umkristallisiert werden. Fällt nach der Reaktion kein Niederschlag aus, wird das Lösungsmittel abgedampft und der Rückstand wird kristallisiert und gegebenenfalls umkristallisiert oder durch Chromatographie gereinigt.

In einer anderen bevorzugten Ausführungsform wird ein flüssiges primäres oder sekundäres Amin bei Raumtemperatur gleichzeitig als Reaktant und als Verdünnungsmittel vorgelegt und mit Ammoniumisocyanat unter Rühren versetzt. Nach beendeter Zugabe wird die Reaktionsmischung zur Vervollständigung der Reaktion erst bei Raumtemperatur und dann bei erhöhter Temperatur gerührt. Nach beendeter Reaktion wird das Amin abdestilliert oder chromatographisch entfernt und der Rückstand gegebenenfalls durch Kristallisieren und Umkristallisieren oder chromatographisch gereinigt.

Das Verfahren ermöglicht eine einfache Herstellung von N-mono- oder N,N-disubstituierten Harnstoffen aus Ammoniumisocyanat in guten Ausbeuten und stellt daher eine Bereicherung der Technik dar.

### Beispiel 1

10,8 g Ammoniumisocyanat (83%ig) (0,15 Mol) wurden in 90 ml Chloroform suspendiert, mit 28,1 g (0,15 Mol) Dodecylamin, gelöst in Chloroform bei Raumtemperatur versetzt, 1 Stunde gerührt und anschließend 2 Stunden auf Rückfluß erhitzt.
Nach dem Abkühlen wurde der ausgefallene Niederschlag abgesaugt und getrocknet.
Dabei wurden 33,5 g Dodecylharnstoff, das sind 98 % der Theorie mit einem Schmelzpunkt von 102 bis 105 °C erhalten.

### Beispiel 2

Auf die im Beispiel 1 beschriebene Art und Weise, aber unter Verwendung von 14,9 g Cyclohexylamin (0,15 Mol) anstatt von Dodecylamin, wurden 20,1 g Cyclohexylharnstoff, das sind 94 % der Theorie erhalten.

| | theoretisch | gefunden |
|---|---|---|
| C (%) | 59,1 | 59,2 |
| H (%) | 9,9 | 9,9 |
| N (%) | 19,7 | 19,7 |

### Beispiel 3

Auf die im Beispiel 1 beschriebene Art und Weise, aber unter Verwendung von 8,9 g Isopropylamin (0,15 Mol) anstatt von Dodecylamin, wurden 13,7 g Isopropylharnstoff, das sind 89 % der Theorie erhalten.

| | theoretisch | gefunden |
|---|---|---|
| C (%) | 46,7 | 47,0 |
| H (%) | 9,8 | 9,6 |
| N (%) | 27,2 | 27,7 |

### Beispiel 4

Auf die im Beispiel 1 beschriebene Art und Weise, aber unter Verwendung von 4,5 g Ethylendiamin (0,075 Mol) anstatt von Dodecylamin, wurden 6,1 g Ethylendiharnstoff, das sind 55 % der Theorie erhalten.

| | theoretisch | gefunden |
|---|---|---|
| C (%) | 32,7 | 32,7 |
| H (%) | 6,9 | 7,0 |
| N (%) | 38,1 | 38,2 |

### Beispiel 5

Auf die im Beispiel 1 beschriebene Art und Weise, aber unter Verwendung von 14,0 g Anilin (0,15 Mol) anstatt Dodecylamin und Wasser anstatt Chloroform, wobei das Reaktionsprodukt nach beendeter Reaktion direkt in der Reaktionsmischung nach Zusatz von Wasser umkristallisiert wurde, wurden 4,9 g Phenylharnstoff, das sind 24% der Theorie mit einem Schmelzpunkt von 145 - 148 °C erhalten.

### Beispiel 6

Auf die im Beispiel 1 beschriebene Art und Weise, aber unter Verwendung von 14,0 g Anilin (0,15 Mol) anstatt Dodecylamin, wurden 7,6 g Phenylharnstoff, das sind 37 % der Theorie, mit einem Schmelzpunkt von 145 - 148 °C erhalten.

### Beispiel 7

18,6 g Anilin (0,2 Mol) wurden unter Rühren bei Raumtemperatur mit 13,3 g Ammoniumisocyanat (90%ig) (0,2 Mol) versetzt. Die Reaktionsmischung wurde eine Stunde bei Raumtemperatur und danach 5 Stunden bei 55 bis 60 °C gerührt. Nach beendeter Reaktion wurde das überschüssige Anilin abdestilliert und der Rückstand aus Wasser umkristallisiert.
Dabei wurden 12,7 g Phenylharnstoff, das sind 47 % der Theorie, mit einem Schmelzpunkt von 145 - 148 °C erhalten.

### Beispiel 8 - 9

20 g Ammoniumisocyanat (90%ig) (0,3 Mol) wurden in 200 g Verdünnungsmittel bei Raumtemperatur suspendiert oder gelöst und mit einer Lösung von 18,0 g 1,6-Diaminohexan (0,15 Mol), gelöst in dem gleichen Verdünnungsmittel unter Rühren versetzt.
Das Reaktionsgemisch wurde eine Stunde bei Raumtemperatur und 5 Stunden bei 55 bis 60 °C gerührt und abgekühlt. Der ausgefallene Niederschlag wurde abgesaugt, aus Wasser umkristallisiert und getrocknet.
Als Verdünnungsmittel wurden die in Tabelle 1 angeführten Verdünnungsmittel eingesetzt. Bei der Verwendung von Wasser als Verdünnungsmittel wurde die Reaktionsmischung nach beendeter Reaktion mit 600 ml Wasser versetzt und der ausgefallene Niederschlag darin umkristallisiert.
Dabei wurden die in Tabelle 1 angeführten Ausbeuten an 1,6-Hexandiharnstoff mit einem Schmelzpunkt von 202 - 205 °C erhalten.

Auf die oben beschriebene Art und Weise, aber unter Zugabe von 32,2 g Benzylamin (0,3 Mol) anstatt von 1,6-Diaminohexan wurden die in Tabelle 1 angegebenen Ausbeuten an Benzylharnstoff erhalten.

| | theoretisch | gefunden |
|---|---|---|
| C (%) | 64,0 | 64,0 |
| H (%) | 6,7 | 6,7 |
| N (%) | 18,7 | 18,7 |

**Tabbelle 1**

| Verdünnungsmittel | **% der theorethischen Ausbeute an** | |
|---|---|---|
| | 1,6-Hexandiharnstoff | Benzylharnstoff |
| n-Hexan | 85 | 76 |
| Toluol | 73 | 67 |
| Chlorbenzol | 56 | 56 |
| Tetrahydrofuran | 71 | 79 |
| Dimethoxyethan | 72 | - |
| 1,4-Dioxan | 74 | 72 |
| Ethanol | 20 | - |
| Wasser | 59 | 56 |

### Beispiel 10

18,0 g 1,6-Diaminohexan (0,15 Mol) wurden in 200 ml Wasser vorgelegt und unter Rühren auf a) 50 °C und b) 100 °C erhitzt. 20,0 g Ammoniumisocyanat (90%ig) (0,3 Mol) wurden in Wasser gelöst und jeweils bei den angegebenen Temperaturen unter Rühren zugetropft. Nach beendeter Reaktion wurde das Reaktionsgemisch mit 600 ml Wasser versetzt und der ausgefallene Niederschlag darin umkristallisiert, abgesaugt und getrocknet.
Dabei wurden a) 45 % und b) 47 % Ausbeute der Theorie 1,6-Hexandiharnstoff mit einem Schmelzpunkt von 202 - 205 °C erhalten.

### Beispiel 11

14,11 g Ammoniumisocyanat (85%ig) (0,2 Mol) wurden in 200 ml n-Hexan suspendiert und mit 48,3 g Dioctylamin (0,2 Mol), gelöst in 200 ml n-Hexan versetzt. Nach beendeter Zugabe wurde eine Stunde bei Raumtemperatur nachgerührt und anschließend zwei Stunden auf Rückfluß erhitzt. Nach dem Abkühlen der Reaktionsmischung fiel etwas Harnstoff aus, der abfiltriert wurde. Das Filtrat wurde bei 100 °C, 0,03 bis 0,04 mbar einer Destillation unterworfen, wobei flüchtige Bestandteile entfernt wurden.

Dabei wurden 51,0 g N,N-Dioctylharnstoff, das sind 90 % der Theorie, in Form eines gelblichen Öles erhalten.

| | theoretisch | gefunden |
|---|---|---|
| C (%) | 71,8 | 71,9 |
| H (%) | 12,8 | 12,9 |
| H (%) | 9,8 | 9,8 |

## Patentansprüche

1. Verfahren zur Herstellung N-mono- oder N,N-disubstituierter Harnstoffe, dadurch gekennzeichnet, daß Ammoniumisocyanat in einem Verdünnungsmittel mit einem primären oder sekundären Amin umgesetzt wird.

2. Verfahren nach Anspruch 1, dadurch gekennzeichnet, daß als Verdünnungsmittel ein unter den Reaktionsbedingungen inertes Verdünnungsmittel eingesetzt wird.

3. Verfahren nach einem der Ansprüche 1 oder 2, dadurch gekennzeichnet, daß als inertes Verdünnungsmittel ein aliphatischer oder aromatischer Kohlenwasserstoff, ein chlorierter, aliphatischer oder aromatischer Kohlenwasserstoff, ein Ether, Alkohol oder Wasser eingesetzt wird.

4. Verfahren nach Anspruch 1, dadurch gekennzeichnet, daß das primäre oder sekundäre Amin gleichzeitig als Verdünnungsmittel eingesetzt wird.

5. Verfahren nach Anspruch 1, dadurch gekennzeichnet, daß Ammoniumisocyanat und das primäre oder sekundäre Amin in äquivalenten Mengen eingesetzt werden, sofern das Amin nicht gleichzeitig als Verdünnungsmittel eingesetzt wird.

6. Verfahren nach Anspruch 1, dadurch gekennzeichnet, daß Ammoniumisocyanat in einem inerten Verdünnungsmittel vorgelegt und unter Rühren bei Temperaturen von -20 °C bis Raumtemperatur mit einem primären oder sekundären Amin versetzt und reagieren gelassen wird, worauf die Temperatur zur Vervollständigung der Reaktion bis auf Rückflußtemperatur des vorgelegten, inerten Verdünnungsmittels erhöht wird.

7. Verfahren nach Anspruch 1, dadurch gekennzeichnet, daß ein primäres oder sekundäres Amin mit oder ohne inertes Verdünnungsmittel vorgelegt und unter Rühren bei Temperaturen von -20 °C bis Raumtemperatur mit Ammoniumisocyanat versetzt und reagieren gelassen wird, worauf die Reaktionstemperatur zur Vervollständigung der Reaktion erhöht wird.

8. Verfahren nach einem der Ansprüche 1 bis 7, dadurch gekennzeichnet, daß der gebildete N-mono- oder N,N-disubstituierte Harnstoff auf übliche Weise aus dem Reaktionsgemisch isoliert wird.

## Claims

1. Process for the preparation of N-mono- or N,N-di-substituted ureas, characterized in that ammonium isocyanate is reacted with a primary or secondary amine in a diluent.

2. Process according to claim 1, characterized in that a diluent which is inert under the reaction conditions is employed as the diluent.

3. Process according to one of claims 1 or 2, characterized in that an aliphatic or aromatic hydrocarbon, a chlorinated aliphatic or aromatic hydrocarbon, an ether, alcohol or water is employed as the inert diluent.

4. Process according to claim 1, characterized in that the primary or secondary amine is employed simultaneously as the diluent.

5. Process according to claim 1, characterized in that ammonium isocyanate and the primary or secondary amine are employed in equivalent amounts, provided that the amine is not employed simultaneously as the diluent.

6. Process according to claim 1, characterized in that ammonium isocyanate is initially introduced into the reaction vessel in an inert diluent, and a primary or secondary amine is added at temperatures of -20°C to room temperature, while stirring, and the mixture is allowed to react, after which the temperature is increased to the reflux temperature of the inert diluent initially introduced, in order to bring the reaction to completion.

7. Process according to claim 1, characterized in that a primary or secondary amine is initially introduced into the reaction vessel with or without an inert diluent, ammonium isocyanate is added at temperatures of -20°C to room temperature, while stirring, and the mixture is allowed to react, after which the reaction temperature is increased in order to bring the reaction to completion.

8. Process according to one of claims 1 to 7, characterized in that the N-mono- or N,N-disubstituted urea formed is isolated from the reaction mixture in a customary manner.

## Revendications

1. Procédé de préparation d'urées N-monosubstituées ou N,N-disubstituées, caractérisé en ce qu'on fait réagir l'isocyanate d'ammonium dans un diluant avec une amine primaire ou secondaire.

2. Procédé selon la revendication 1, caractérisé en ce qu'on utilise, comme diluant, un diluant qui est inerte dans les conditions de réaction.

3. Procédé selon la revendication 1 ou 2, caractérisé en ce qu'on utilise, comme diluant inerte, un hydrocarbure aliphatique ou aromatique, un hydrocarbure aliphatique ou aromatique chloré, un éther, un alcool ou de l'eau.

4. Procédé selon la revendication 1, caractérisé en ce que l'amine primaire ou secondaire utilisée sert en même temps de diluant.

5. Procédé selon la revendication 1, caractérisé en ce qu'on utilise des quantités équivalentes d'isocyanate d'ammonium et d'amine primaire ou secondaire, à la condition que l'amine ne serve pas en même temps de diluant.

6. Procédé selon la revendication 1, caractérisé en ce qu'on introduit l'isocyanate d'ammonium dans un diluant inerte, on y ajoute une amine primaire ou secondaire, sous agitation, à des températures comprises entre -20°C et la température ambiante, et on laisse réagir le mélange résultant, après quoi, pour la réalisation complète de la réaction, on augmente la température jusqu'à la température de reflux du diluant inerte introduit préalablement.

7. Procédé selon la revendication 1, caractérisé en ce qu'on introduit une amine primaire ou secondaire, avec ou sans diluant inerte, on y ajoute de l'isocyanate d'ammonium, sous agitation, à des températures comprises entre -20°C et la température ambiante, et on laisse réagir le mélange résultant, après quoi on augmente la température pour la réalisation complète de la réaction.

8. Procédé selon l'une des revendications 1 à 7, caractérisé en ce qu'on isole l'urée N-monosubstituée ou N,N-disubstituée du mélange de réaction d'une manière usuelle.
